# EUROPEAN PATENT APPLICATION

(11) **EP 2 502 995 A1**
(43) Date of publication of application: **26.09.2012**
(21) Application number: 10831655.5
(22) Date of filing: 19.11.2010
(51) Int. Cl.: C12N 15/09, A61K 39/00, A61K 45/00, A61P 31/12, C07K 16/10

(54) **PHARMACEUTICAL COMPOSITION**

(30) Priority: 20.11.2009 JP 2009265390
(71) Applicant: Tokyo Metropolitan Institute of Medical Science, Tokyo 156-8506 (JP)
(72) Inventor: SHIBASAKI, Futoshi, Tokyo 156-8506 (JP); SAKURAI, Akira, Tokyo 156-8506 (JP)
(74) Representative: Denison, Christopher Marcus
(86) International application number: PCT/JP2010/070713
(87) International publication number: WO 2011/062263

(57) **Abstract**

The present invention is directed to provide infection inhibitor products for an influenza virus. More specifically, an inhibitory substance that inhibits the activity of an RR sequence to permit a molecule of which it forms a part to be introduced into cells, such as an antibody that recognizes a peptide having an amino acid sequence RERRRKKR (SEQ ID NO. 1), is formulated as a pharmaceutical composition and an infection inhibitor products for an influenza virus containing the pharmaceutical composition is manufactured.

## Description

### Cross Reference to Related Applications

This application claims the benefit of Japanese Patent Application No. 2009-26539 filed on November 20, 2009, the entire disclosure of which is hereby incorporated by reference.

### TECHNICAL FIELD

The present invention relates to pharmaceutical compositions.

### BACKGROUND ART

Influenza viruses are classified into types A, B and C. Of these, influenza A viruses affect both humans and animals. Type A viruses are further classified into various subtypes based on the combinations of 16 types of their surface antigen hemagglutinin (HA) and 9 types of neuraminidase (NA). These viruses are distinguished as low pathogenic and highly pathogenic forms on the basis of their HA antigenicity. Low pathogenic viruses make only local infection (e.g., respiratory infection or intestinal infection) while highly pathogenic H5 and H7 viruses infect and replicate systemically in multiple organs and may kill their host.

Infection of cells with an influenza virus requires binding of HA to a receptor on the host cell surface. After the influenza virus has bound to the receptor, it is taken up by cells and then begins to replicate within the cells. Low pathogenic viruses require cleavage of HA by an extracellular protease that is secreted locally to obtain infectivity, which indicates that the cleavage of HA is coupled to the infection. On the contrary, highly pathogenic viruses replicate within cells; and their HA is cleaved and thus they become infectious when they are released from the cell. This means that the highly pathogenic viruses that are present outside the cell already retain infectivity and the cleavage of HA is not coupled to the infection. Accordingly, the HA of highly pathogenic viruses is said to be of a self-cleaved type (Kawaoka, Y. and Webster, R. G., Proc. Natl. Acad. Sci. USA, vol. 85, pp. 324-328, 1988).

Most subtypes of influenza viruses infect or bind to receptors in a species-specific manner, so that avian influenza viruses do not typically infect humans. However, it is considered that mutations in the avian influenza viruses and the resultant alteration of receptor recognition could confer a high risk of human infections with such viruses.

Although transmission of avian influenza viruses from birds to humans has been reported in some cases, human-to-human transmission of these viruses has not yet been observed. However, an outbreak of avian influenza is threatened if the highly pathogenic avian influenza viruses acquire the ability to transmit to humans, because humans do not have immunity to this kind of viruses. Such a situation creates a demand for development of a prophylactic or therapeutic drug for the highly pathogenic influenza viruses.

### DISCLOSURE OF THE INVENTION

An object of the present invention is to provide infection inhibitor products for influenza viruses.

Until now, it has been unclear how highly pathogenic influenza viruses can invade the cells throughout the body. Expression pattern of the protease for the cleavage cannot account for the systemic cell infections of the highly pathogenic influenza viruses because these viruses have already experienced HA cleavage in the extracellular environment.

The highly pathogenic influenza viruses are known to possess a specific series of basic amino acids, RERRRKKR (SEQ ID NO. 1), at the cleavage site of HA. However, the function of this sequence has not yet been elucidated. The present inventors investigated the function of this amino acid sequence and became aware of the fact that a peptide with this amino acid sequence could serve as a cell penetrating peptide (CPP). This function is expected to be responsible for the systemic infection of the highly pathogenic influenza viruses. Taken the above into consideration, the present inventors produced a polyclonal antibody that recognozes the amino acid sequence in question and found that this antibody could inhibit the entry of the peptide with this amino acid sequence into cells. In this way, it was revealed that the antibody that recognozes this amino acid sequence can suppress the infection with the highly pathogenic influenza viruses, and the present invention was thus completed.

An aspect of the present invention is an antibody or a fragment thereof that recognizes a peptide having an RR sequence, in particular, an amino acid sequence RERRRKKR (SEQ ID NO. 1), or an expression vector that expresses at least one of them. It is preferable that said antibody or a fragment thereof recognize the RR sequence, in particular, the amino acid sequence of RERRRKKR (SEQ ID NO. 1) as an epitope. In addition, said antibody or a fragment thereof may be a polyclonal antibody or a monoclonal antibody.

Another aspect of the present invention is a pharmaceutical composition containing an inhibitory substance that inhibits the activity of an RR sequence, in particular, RERRRKKR (SEQ ID NO. 1) to permit a peptide or a molecule to be introduced into cells, the RR sequence forming a part of the molecule, and an entry inhibitor product that inhibits the entry of the peptide having the RR sequence, in particular, the amino acid sequence of RERRRKKR (SEQ ID NO. 1) into cells. Said inhibitory substance may be said antibody or a fragment thereof, or an expression vector that expresses one of them. Said highly pathogenic influenza virus may be the H5N1 subtype of the influenza A virus.

A yet another aspect of the present invention is an infection inhibitor product for a highly pathogenic influenza virus that inhibits infection of a cell by the highly pathogenic influenza virus, or a medicament for preventively, prophylactically or curatively treating influenza caused by the highly pathogenic influenza virus in which the medicament contains the inhibitory substance that inhibits the activity of an RR sequence, in particular, RERRRKKR (SEQ ID NO. 1) to permit a molecule to be introduced into cells, the RR sequence forming a part of the molecule. It is preferable that said cell have no functional influenza virus receptor, and especially, it is preferable that said cell be other than a respiratory or intestinal cell. If the cell is a respiratory or intestinal cell, it is preferable that the function of the influenza virus receptor be inhibited. The inhibitory substance may be said antibody or a fragment thereof, or an expression vector that expresses one of them. The highly pathogenic influenza virus may be the H5N1 subtype of the influenza A virus.

Another aspect of the present invention is a method for preventively, prophylactically or curatively treating highly pathogenic influenza, comprising the step of administering an inhibitory substance that inhibits the activity of an RR sequence, in particular, RERRRKKR (SEQ ID NO. 1) to permit a molecule to be introduced into cells, the RR sequence forming a part of the molecule, to a healthy person or an influenza patient infected with a highly pathogenic influenza virus. The inhibitory substance may be said antibody or a fragment thereof, or an expression vector that expresses one of them. The highly pathogenic influenza virus may be the H5N1 subtype of the influenza A virus. In addition, the inhibitory substance may be administered to the patient while blocking an infection route for a low pathogenic influenza virus. For example, a neuraminidase inhibitor may be used along with the inhibitory substance. It is preferable that said neuraminidase inhibitor is selected from the group consisting of oseltamivir, zanamivir, salts thereof, hydrates thereof, and mixtures thereof.

### BRIEF DESCRIPTION OF THE DRAWINGS

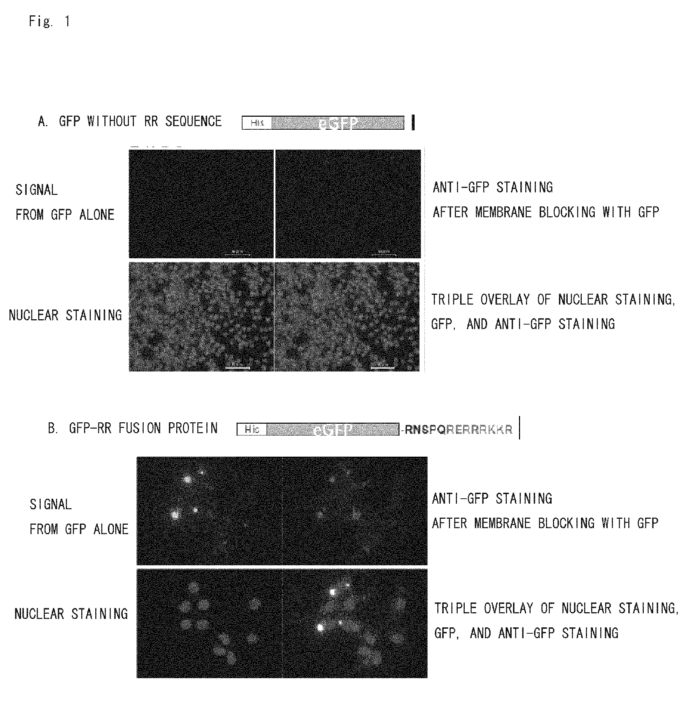

Fig. 1 shows that, in one example of the present invention, a peptide having an RR sequence enters cells. In both Figs. 1A and 1B, the upper left panel represents GFP fluorescence signals, the upper right panel represents signals obtained by antibody staining with an anti-GFP antibody, the lower left panel represents signals obtained by nuclear staining with DAPI, and the lower right panel represents a triple overlay of GFP fluorescence signals, signals obtained by antibody staining with an anti-GFP antibody, and signals obtained by nuclear staining with DAPI.

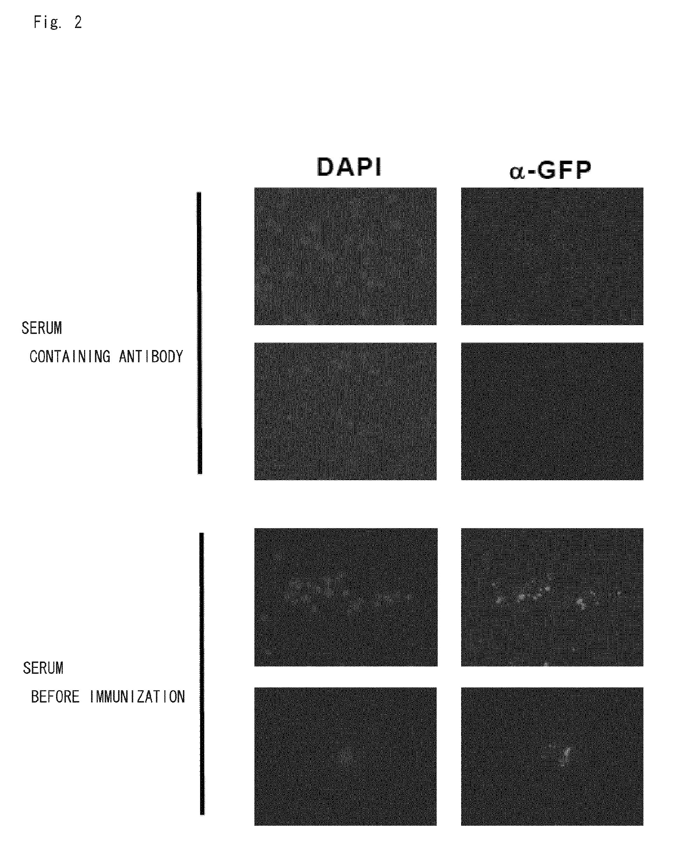

Fig. 2 shows that, in one example of the present invention, a polyclonal antibody that recognizes a peptide having an RR sequence can inhibit the entry of that peptide into cells. The four panels in the left column represent signals obtained by nuclear staining with DAPI while the four panels in the right column represent signals obtained by antibody staining with an anti-GFP antibody. The upper four panels correspond to experiments using a GFP-RR fusion protein treated with a polyclonal antibody that recognizes a peptide having an RR sequence while the lower four panels correspond to control experiments using a GFP-RR fusion protein treated with rabbit serum before immunization.

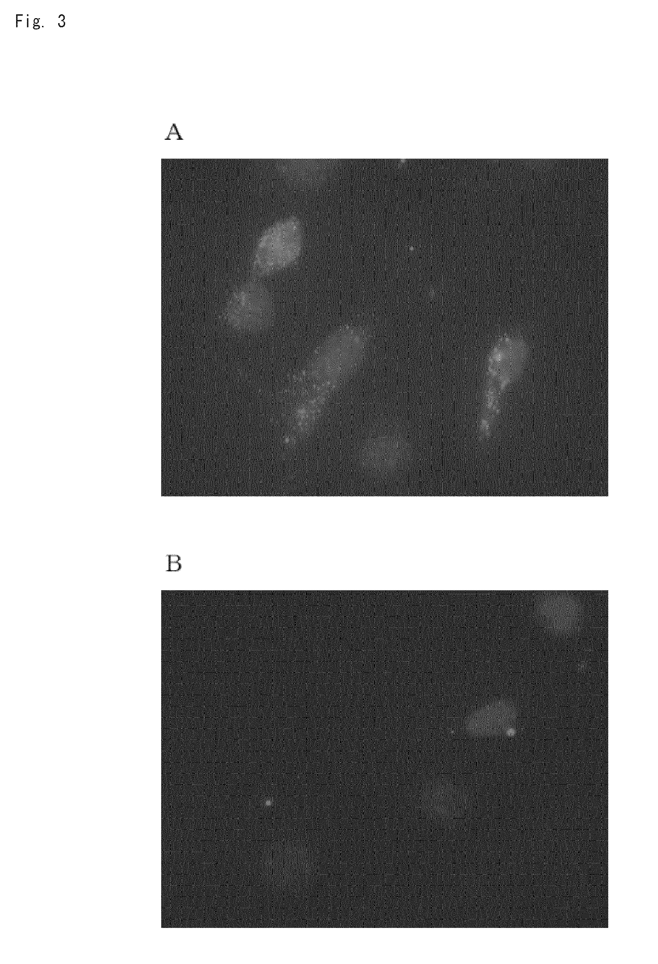

Fig. 3 shows that, in one example of the present invention, a Fab fragment of a polyclonal antibody that recognizes a peptide having an RR sequence can inhibit the entry of that peptide into cells. Fig. 3A corresponds to a control experiment using a GFP-RR fusion protein treated with rabbit serum before immunization while Fig. 3B corresponds to an experiment using a GFP-RR fusion protein treated with a polyclonal antibody that recognizes a peptide having an RR sequence.

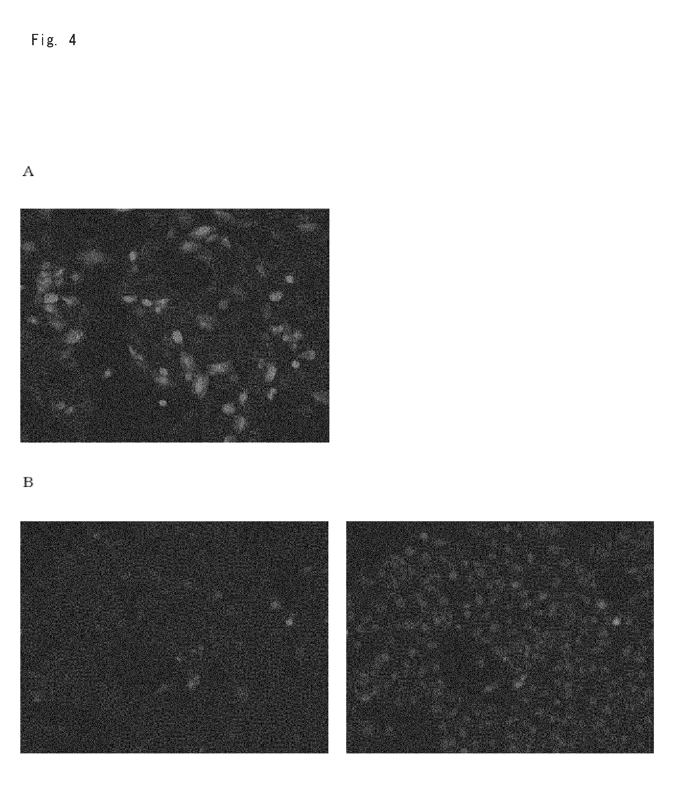

Fig. 4 shows that, in one example of the present invention, when cells were infected with a highly pathogenic influenza virus after the cell surface sialic acid was removed by sialidase treatment, an antibody specific for an RR sequence has an effect of inhibiting the infection. Fig. 4A corresponds to an experiment in which cells were infected with a highly pathogenic influenza virus treated with PBS while Fig. 4B corresponds to an experiment in which cells were treated with serum containing the antibody specific for the RR sequence. The right panel in Fig. 4B is a photograph of nuclei of the cells stained with DAPI.

### MODE FOR CARRYING OUT THE INVENTION

Unless otherwise noted in embodiments and examples, all procedures used are as described in standard protocols such as J. Sambrook, E. F. Fritsch & T. Maniatis (Ed.), Molecular cloning, a laboratory manual (3rd edition), Cold Spring Harbor Press, Cold Spring Harbor, New York (2001); F. M. Ausubel, R. Brent, R. E. Kingston, D. D. Moore, J.G. Seidman, J. A. Smith, K. Struhl (Ed.), Current Protocols in Molecular Biology, John Wiley & Sons Ltd., with or without modifications or changes. In addition, unless otherwise noted, a commercial reagent kit or a measurement instrument, if any, is used as described according to protocols attached thereto.

The above and further objects, features, advantages, and ideas of the present invention are apparent from those skilled in the art from consideration of the detailed description of this specification. Furthermore, those skilled in the art can easily reproduce the present invention from these descriptions. The mode(s) and specific example(s) described below represent a preferable embodiment of the present invention, which is given for the purpose of illustration or description. The present invention is not limited thereto. It is obvious to those skilled in the art that various modifications may be made according to the descriptions of the present specification without departing from the spirit and scope of the present invention disclosed herein.

An embodiment of the present invention is an inhibitory substance that inhibits the activity of an RR sequence, in particular, RERRRKKR (SEQ ID NO. 1) to permit a molecule of which it forms a part to be introduced into cells. This inhibitory substance is preferably an antibody that recognizes a peptide having the RR sequence (hereinafter, referred to as an "RR peptide recognition antibody"). However, the inhibitory substance is not specifically limited and may be an aptamer, a cyclic peptide, or a low molecular compound, as long as it can inhibit the activity of the RR sequence to permit a molecule of which it forms a part to be introduced into cells. The antibody is not specifically limited as long as it has a structure to inhibit the function of the RR sequence. However, it is preferable that the antibody recognizes the RR sequence as an epitope. This antibody may be monoclonal or polyclonal, or may be an artificial antibody such as a humanized antibody. These antibodies can be produced by any production method known to those skilled in the art. The antigen used for this purpose may be the RR sequence itself or may be conjugated with another substance such as hapten.

It is noted that the RR sequence refers to a basic sequence of amino acids consisting of 5-8 amino acid residues, located at the cleavage site of HA of highly pathogenic influenza viruses. More specifically, the RR sequence includes a sequence RERRRKKR (SEQ ID NO. 1) of the H5N1 subtype of the influenza A virus, variants thereof (SEQ ID NOS. 2-4) and basic amino acid sequences (SEQ ID NOS. 5-9) of the H7 subtype of the influenza A virus (see Table 1).

**Table 1**

| INFLUENZA VIRUS TYPE OF DNA SEQUENCE | SEQUENCE | SEQ ID NO. |
|---|---|---|
| H5 Common sequence | RERRRKKR | 1 |
| H5 Indonesia | RESRRKKR | 2 |
| H5 Egypt | GERRRKKR | 3 |
| H5 China | RERRRKR | 4 |
| H7 | KRRRR | 5 |
| | KKREKR | 6 |
| | KKRRKR | 7 |
| | KKKKKKKR | 8 |
| | HKQLTHHMRKKR | 9 |

Another embodiment of the present invention may be a fragment of the RR peptide recognition antibody. This antibody fragment is not specifically limited as long as it is a part of an antibody and is an antigen binding site containing the variable region. As an example, a Fab fragment or a F(ab')₂ fragment may be used. These fragments can be obtained by, for example, partial digestion of a monoclonal antibody with a protease. Any protease may be used, provided that it can produce Fab fragments or F(ab')₂ fragments. Examples include enzymes such as pepsin and ficin.

Yet another embodiment provides DNA that codes for the RR peptide recognition antibody or a fragment thereof, or an expression vector having the DNA that expresses the RR peptide recognition antibody or a fragment thereof. The DNA and the expression vector can be produced by any production method known to those skilled in the art.

The RR peptide recognition antibody or a fragment thereof may be produced by constructing an expression vector using a promoter that functions in, for example, *E*. *coli* or mammalian cultured cells, introducing the expression vector into the host cells to express the antibody or a fragment thereof, and then purifying it. It is preferable that a signal peptide is added to the antibody or a fragment thereof, which is secreted outside the cell.

An RR sequence inhibitory substance that inhibits the activity of the RR sequence, in particular, RERRRKKR (SEQ ID NO. 1) to permit a molecule of which it forms a part to be introduced into cells, preferably, the RR peptide recognition antibody or a fragment thereof can bind to a peptide having the RR sequence and inhibit interactions between a cell membrane and the site containing the RR sequence, and thereby can inhibit the entry of this peptide into cells. Accordingly, the RR sequence inhibitory substance can be used *in vivo* and *in vitro* as an entry inhibitor product that inhibits the entry of the peptide having the RR sequence into cells. Likewise, the expression vector that can secrete the RR peptide recognition antibody or a fragment thereof outside the cell may also be used as the entry inhibitor product. The cell is not limited to a specific one. It is preferable, however, that the cell is not infected with any low pathogenic influenza viruses but is infected with highly pathogenic influenza viruses, such as the cell having no functional influenza virus receptor. A cell other than a respiratory or intestinal cell is particularly preferable. If the cell is a respiratory or intestinal cell, it is preferable that the function of the influenza virus receptor is inhibited. The functional influenza virus receptor as used herein refers to a receptor to which a low pathogenic influenza virus binds to infect the cells, that is, a receptor having a carbohydrate structure with a sialic acid on the cell surface, to which the influenza viruses binds. How to inhibit its function is not specifically limited. For example, sialic acid on the cell surface may be removed by sialidase treatment, or a compound that binds to sialic acid competitively with the influenza virus may be administered.

In addition, highly pathogenic influenza viruses have an RR sequence at the terminus of the capsid peptide and enter cells with this sequence. Taken this into consideration, the RR sequence inhibitory substance is intended to bind to the capsid peptide to inhibit the highly pathogenic influenza viruses from entering the cells. Thus, the RR sequence inhibitory substance can be used as an infection inhibitor product for highly pathogenic influenza viruses.

The highly pathogenic influenza viruses as used herein are not specifically limited as long as they systemically infect the host. These viruses preferably have the RR sequence at the cleavage site of HA. It is also preferable that when these viruses replicate in the cells and are released from the cells, their HA is already cleaved and the capsid peptide has the RR sequence at the terminus thereof. More specifically, examples include the influenza virus with H5 and H7 subtypes of HA

As described above, the RR sequence inhibitory substance can inhibit the entry of the highly pathogenic influenza virus into cells, so that it can be used as a pharmaceutical composition for diseases caused by highly pathogenic influenza viruses. A target subject to which the pharmaceutical composition is to be administered may be any vertebrate and is not specifically limited. However, it is preferable that the subject be humans, birds, or pigs.

This pharmaceutical composition may be formulated into a dosage form such as tablets, powders, granules, microspheres, capsules, liquids, emulsions, and suspensions, with the addition of a pharmaceutically acceptable carrier, if necessary. With this, it is possible to produce a medicament for preventively, prophylactically or curatively treating influenza. The pharmaceutically acceptable carrier as used herein may appropriately be selected from carriers that are normally used, depending on the medicament to be prepared. For example, when the medicament is prepared as a solution, the carrier may be purified water (sterile water), physiological buffer, or injectable organic esters such as glycol, glycerol, and olive oil. The medicament may contain additives that are normally used, such as stabilizers and excipients. The administration route is not specifically limited and may be determined appropriately depending on, for example, dosage forms, age and sex of a patient, seriousness of the disease, and other conditions. In particular, it is preferable that the dosage form be those for parenteral administration, such as injectables, infusions, and aerosols. When the medicament is used as an injectable or infusion, it is mixed with salt solution or normal fluid such as a glucose or amino acid solution, if necessary and then injected intravenously, intramuscularly, intradermally, subcutaneously or intraperitoneally. This medicament may be used alone or in combination with other drug(s) (such as other antiviral drug(s), anti-inflammatory drug(s), drug(s) for alleviating symptoms). In particular, it is preferable that the medicament be administered while blocking an infection route for a low pathogenic influenza virus. For example, it is preferable that the medicament be used along with a neuraminidase inhibitor such as oseltamivir or salts or hydrates thereof (e.g., an oseltamivir phosphate, Tamiflu® (Roche)) and zanamivir or salts or hydrates thereof (e.g., a zanamivir hydrate, Relenza (GlaxoSmithKline)), both of which are an inhibitor against such infection.

When combined, the RR sequence inhibitory substance may be administered simultaneously with a neuraminidase inhibitor. Alternatively, they may be administered sequentially within a period during which the action of the previously administered one lasts.

### EXAMPLES

### (1) Synthesis of fusion protein of GFP protein and RR sequence (hereinafter, referred to as "GFP-RR fusion protein")

Using DNA obtained from highly pathogenic avian influenza Vietnam1203 H5N1, RR sequence was amplified by PCR using the following primers.
forward: ACCATTggggAATgCCCCAAATATgTgAAATC (SEQ ID NO. 10) reverse: CgACAAgCTTgAATTCTTATCTCTTTTTTCTTCTTCTCTC (SEQ ID NO. 11)

A combination of this sequence and an EGFP gene or the EGFP gene alone was inserted into the protein expression vector pCold-II (Takara). The EGFP was amplified using the following primers.
EGFP primer-F: CgAgggATCCgAATTCAgTAAAggAgAAgAACTTTTCAC (SEQ ID NO. 12)
EGFP primer-R: gCATTCCCCAATggTTTTgTATAgTTCATCCATgCCATg (SEQ ID NO. 13)

The vector pCold-II was cleaved with EcoRI. The DNA was inserted into it by homologous recombination using an In-fusion kit (Takara). The GFP portion was fused at the N terminus of the RR sequence in GFP-RR. This expression vector was introduced into *E*. *coli* host strains, Rosseta-Gami™ (Novagen), and cultured at 15°C for 48 hours. Cultured cells were lysed with BugBuster® (Novagen) and then the protein was purified using Ni-Sepharose™ (GE Healthcare).

### (2) Entry of GFP-RR fusion protein into cells

Ten micrograms of GFP-RR fusion protein or GFP protein without the RR sequence was added to a medium (a 1:9 mixture of D-MEM with 10% FCS and OPTI-MEM) of BHK cells (1 x 10⁵ cells/ml) and incubated in the presence of 5% CO₂ at 37°C for 3 hours. The cells were fixed with paraformaldehyde and blocked with skimmed milk containing an anti-GFP antibody (polyclonal, MBL, 500 x dilution) for 30 minutes at 37°C. Subsequently, the cells were treated with 0.1% Triton X-100 for 30 minutes at room temperature. They were immunostained with an anti-GFP antibody (monoclonal, MBL, 500 x dilution) used as the primary antibody for 30 minutes at 37°C, and with a Cy3-conjugated anti-mouse IgG antibody (polyclonal, Jackson, 500 x dilution) used as the secondary antibody for 30 minutes at 37°C. The entry of GFP-RR fusion protein into the cells was examined based on the presence of Cy3 fluorescence. At the same time, the fluorescence of GFP was detected. Nuclei were stained with DAPI and the fluorescence of DAPI was also detected.

No fluorescence was detected for Cy3 and GFP when the GFP protein without the RR sequence was used (Fig. 1A), indicating that this protein did not enter the cells. On the contrary, fluorescences for Cy3 and GFP were detected in the cells when the GFP-RR fusion protein was used (Fig. 1B), indicating that this fusion protein has entered the cells. The nuclei stained with DAPI are shown as faint round spots in the four panels in the lower rows in Figs. 1A and 1B. The GFP-RR fusion protein is visualized as discrete bright dots in the three panels in Fig. 1B, except for the one at the lower left.

Thus, the protein with the RR sequence can enter the cells by themselves.

### (3) Production of polyclonal antibody

A rabbit was immunized with a synthetic peptide (Sequence: CTGLRNSPQRERRRRKKR (SEQ ID NO. 14)) four times at two-week intervals. More specifically, 600 µg of the peptide for the primary immunization and 300 µg for the secondary and subsequent immunizations were subcutaneously injected on the back of the rabbit. As adjuvant, used were 300 µl of TiterMax Gold® (Funakoshi) for the primary immunization and incomplete Freund's adjuvant for the secondary and subsequent immunizations. Whole blood was drawn two weeks after the last immunization, and serum was prepared and used as a polyclonal antibody.

### (4) Production of monoclonal antibody

A mouse was immunized with a synthetic peptide (Sequence: CTGLRNSPQRERRRRKKR (SEQ ID NO. 14)) four times at two week intervals. More specifically, 100 µg of the peptide was intraperitoneally or subcutaneously injected for the primary immunization and also for the secondary and subsequent immunizations. As adjuvant, used were 300 µl of TiterMax Gold® (Funakoshi) for the primary immunization and incomplete Freund's adjuvant for the secondary and subsequent immunizations. The mouse was sacrificed two weeks after the last immunization and the spleen was removed. Then, monoclonal antibodies were produced using routine procedures. These antibodies were screened by ELISA with the aforementioned peptide used for the immunization to obtain an antibody that binds to that peptide.

### (5) Inhibition of entry by polyclonal antibody

Ten microliters of rabbit serum taken before or after immunization were added to 10 µg of GFP-RR fusion protein and incubated for 3 hours at room temperature. The mixture was then added to a medium (a 1:9 mixture of D-MEM with 10% FCS and OPTI-MEM) of C1C12 cells (1 x 10⁵/ml) and incubated in the presence of 5% CO₂ at 37°C for 3 hours.

The cells were fixed with paraformaldehyde and blocked with skimmed milk containing an anti-GFP antibody (polyclonal, MBL, 500 x dilution) for 30 minutes at 37°C. Subsequently, the cells were treated with 0.1% Triton X-100 for 30 minutes at room temperature. They were immunostained with an anti-GFP antibody (monoclonal, MBL, 500 x dilution) used as the primary antibody for 30 minutes at 37°C, and with a Cy3-conjugated anti-mouse IgG antibody (polyclonal, Jackson, 500 x dilution) used as the secondary antibody for 30 minutes at 37°C. The entry of GFP-RR fusion protein into the cells was examined based on the presence of Cy3 fluorescence. At the same time, nuclei were stained with DAPI. Fig. 2 shows fluorescence micrographs (two fields each) of the stainings observed.

With rabbit serum taken before immunization, expression of GFP was observed in the cells, indicating that the fusion protein has entered the cells (panels in the lower half of Fig. 2). On the other hand, with rabbit serum containing the antibody after immunization, no GFP expression was observed in the cells, indicating that the entry of the fusion protein into the cells was inhibited (panels in the upper half of Fig. 2). The nuclei stained with DAPI are shown as faint round spots in the four panels in the left column in Fig. 2. The GFP-RR fusion protein is visualized as discrete bright dots in the lower two panels in the right column in Fig. 2.

Thus, the antibody to the RR sequence can inhibit the protein with the RR sequence from entering the cells, so that this antibody can be considered as an inhibitory substance which inhibits the incorporation of the RR sequence into the cells.

### (6) Inhibition of entry by Fab fragment

### 1) Production of Fab fragment

An IgG antibody was purified on Protein G columns from rabbit serum containing antibodies after immunization. More specifically, 18 ml of Tris-HCl buffer (pH = 8.0) was added to 2 ml of serum and the mixture was centrifuged at 15,000 rpm for 10 minutes. The supernatant was mixed with Protein G Sepharose 4 Fast Flow (GE Healthcare). The mixture was loaded onto an open column, washed with 10 ml of PBS/0.1 Tween 20, 20 ml of Tris-HCl buffer (pH = 8.0), and 10 ml of PBS, eluted with 1 ml of 100 mM Glycin-HCl (pH = 3.0), and neutralized by the addition of 1 ml of 1 M Tris-HCl (pH = 9.0).

A Fab fragment was prepared from the purified antibodies using Pierce Fab Preparation Kit (Thermo Fisher Scientific, Inc.) according to the protocol provided with the kit. More specifically, the purified antibodies were digested with papain, and Fc fragments and intact IgG were absorbed on Protein A to purify the Fab fragment.

### 2) Inhibition experiments using Fab fragment

Ten microliters of the Fab fragment were added to 10 µl (10 µg) of the GFP-RR fusion protein and incubated for 3 hours at room temperature. C1C12 cells (1 x 10⁵/ml) were prepared, and 1-ml aliquots were plated on culture dishes of 3.5 cm in diameter. To the medium (a 1:9 mixture of D-MEM with 10% FCS and OPTI-MEM), the whole amount (10 µg) of the GFP-RR fusion protein treated with the Fab fragment was added, and the mixture was incubated for 3 hours at 37°C in the presence of 5% CO₂.

The treated C1C12 cells were fixed with paraformaldehyde and blocked with skimmed milk containing an anti-GFP antibody (rabbit polyclonal, MBL, 500 x dilution) for 30 minutes at 37°C. Subsequently, the cells were treated with 0.1% Triton X-100 for 30 minutes at room temperature. They were immunostained with an anti-GFP antibody (mouse monoclonal, MBL, 500 x dilution) used as the primary antibody for 30 minutes at 37°C, and with a Cy3-conjugated anti-mouse IgG antibody (polyclonal, Jackson, 500 x dilution) used as the secondary antibody for 30 minutes at 37°C. The entry of GFP-RR fusion protein into the cells was examined based on the presence of Cy3 fluorescence. At the same time, nuclei were stained with DAPI. The GFP-RR fusion protein treated with the rabbit serum before immunization was used as a control. Fig. 3 shows fluorescence micrographs of the stainings observed.

As shown in Fig. 3A, when the GFP-RR fusion protein treated with the rabbit serum before immunization was administered to the C1C12 cells (control), expression of GFP was observed in the cells, indicating that the fusion protein has entered the cells. On the other hand, as shown in Fig. 3B, no GFP expression was observed in the cells when the GFP-RR fusion protein treated with the Fab fragment was used, indicating that the entry of the fusion protein into the cells was inhibited. The GFP-RR fusion protein is visualized as discrete bright dots in Fig. 3A while the nuclei stained with DAPI are shown as faint round spots in Figs. 3A and 3B.

Thus, the Fab fragment of the antibody to the RR sequence can also inhibit the protein with the RR sequence from entering the cells, so that this Fab fragment can be used as an inhibitory substance which inhibits the incorporation of the RR sequence into the cells.

### (7) Experiment of infection of cells deficient in sialic acid on their cell membranes with influenza virus

As a model system for administration of a neuraminidase inhibitor product, cells were infected with a highly pathogenic influenza virus after the sialic acid on the cell surface was removed by sialidase treatment. The antibody specific for the RR sequence was used to verify its effect of inhibiting the infection.

Neuraminidase (Seikagaku Kogyo) isolated from Streptococcus 6646K strain was used as the sialidase. First, 0.17 U/ml of enzyme was added to a medium of MDCK cells (2 x 10⁵/ml) and treated for one hour at 37°C to remove the sialic acid on the cell surface. Then, A/Whooper Swan/Hokkaido/2008 (H5N1) highly pathogenic avian influenza virus was added to the medium and the cells were infected with it by MOI = 1. The influenza virus used was preincubated for 1 hour at 37°C in PBS or serum described in the section (3) above.

Eight hours after the infection, the cells were washed with PBS, fixed with a 3.7% formaldehyde solution, and treated with 0.1% Triton X-100 for 30 minutes at room temperature. Subsequently, the cells were treated with an anti-influenza virus NP protein antibody (monoclonal, Millipore Corporation, 500 x dilution) used as the primary antibody for 1 minute at 37°C, and with a Cy3-conjugated anti-mouse IgG antibody (polyclonal, Jackson, 500 x dilution) used as the secondary antibody for 30 minutes at 37°C. Efficacy of virus infection was determined based on the presence of Cy3 fluorescence. Fig. 4 shows fluorescence micrographs of the stainings observed.

The influenza virus treated with PBS has entered the cell even after the removal of the sialic acid on the cell surface (Fig. 4A). On the contrary, when the influenza virus was treated with the serum containing the antibody specific for the RR sequence, virtually no entry of the influenza virus into the cells was detected (left panel in Fig. 4B). For the purpose of visualizing the cells, nuclei were stained with DAPI (right panel in Fig. 4B). Thus, the antibody specific for the RR sequence significantly inhibits the entry of the influenza virus into the cells.

Thus, the antibody to the RR sequence can inhibit the infection of the cells by the highly pathogenic influenza virus using the RR sequence. Accordingly, this antibody is useful as an infection inhibitor product for an influenza virus.

### INDUSTRIAL APPLICABILITY

The present invention makes it possible to provide an infection inhibitor product for an influenza virus.

## Claims

1. An antibody or a fragment thereof which recognizes a peptide having an RR sequence.

2. The antibody or a fragment thereof according to Claim 1, wherein the antibody or a fragment thereof recognizes an amino acid sequence of the RR sequence as an epitope.

3. The antibody or a fragment thereof according to Claim 1 or 2, wherein the RR sequence is RERRRKKR (SEQ ID NO. 1).

4. The antibody or a fragment thereof according to any one of Claims 1 to 3, wherein said antibody is a polyclonal antibody or a monoclonal antibody.

5. A DNA coding for the antibody or a fragment thereof according to any one of Claims 1 to 4.

6. An expression vector, wherein the vector comprises the DNA according to Claim 5 and is capable of expressing the antibody or a fragment thereof according to Claims 1 to 4.

7. A pharmaceutical composition comprising an inhibitory substance, wherein the substance inhibits the activity of an RR sequence to permit a molecule to be introduced into a cell, the RR sequence forming a part of the molecule.

8. The pharmaceutical composition as claimed in Claim 7, wherein said inhibitory substance is the antibody or a fragment thereof claimed in any one of Claims 1 to 4, or the expression vector claimed in Claim 6.

9. A medicament for preventively, prophylactically or curatively treating influenza, comprising the pharmaceutical composition of Claim 7.

10. The medicament for preventively, prophylactically or curatively treating influenza as claimed in Claim 9, wherein said medicament is used with a neuraminidase inhibitor.

11. An entry inhibitor product for inhibiting the entry of a peptide having an RR sequence into cells comprising an inhibitory substance, wherein the substance inhibits the activity of the RR sequence to permit a molecule to be introduced into cells, the RR sequence forming a part of the molecule.

12. The entry inhibitor product according to Claim 11, wherein said inhibitory substance is the antibody or a fragment thereof according to any one of Claims 1 to 4, or the expression vector according to Claim 6.

13. An infection inhibitor product for a highly pathogenic influenza virus for inhibiting infection of a cell by the highly pathogenic influenza virus with an RR sequence, comprising an inhibitory substance, wherein the substance inhibits the activity of the RR sequence to permit a molecule to be introduced into a cell, the RR sequence forming a part of the molecule.

14. The infection inhibitor product for a highly pathogenic influenza virus according to Claim 13, wherein said cell does not have a functional influenza virus receptor.

15. The infection inhibitor product for a highly pathogenic influenza virus according to Claim 14, wherein said cell is other than a respiratory or intestinal cell, or if said cell is a respiratory or intestinal cell, the function of the influenza virus receptor is inhibited.

16. The infection inhibitor product for a highly pathogenic influenza virus according to any one of Claims 13 to 15, wherein said inhibitory substance comprises the antibody or a fragment thereof according to any one of Claims 1 to 4, or the expression vector according to Claim 6.

17. The infection inhibitor product for a highly pathogenic influenza virus according to any one of Claims 13 to 15, wherein said highly pathogenic influenza virus is the H5N1 subtype of the influenza A virus.


SEQUENCE LISTING

<110>  Tokyo Metropolitan Organization for Medical Research

<120>  Pharmaceutical composition

<130>  PCT1813

<150>  JP 2009-26539
<151>  2009-11-20

<160>  14

<170>  PatentIn version 3.5

<210>  1
<211>  8
<212>  PRT
<213>  Influenza virus

<400>  1

Arg Glu Arg Arg Arg Lys Lys Arg
1               5


<210>  2
<211>  8
<212>  PRT
<213>  Influenza virus

<400>  2

Arg Glu Ser Arg Arg Lys Lys Arg
1               5


<210>  3
<211>  8
<212>  PRT
<213>  Influenza virus

<400>  3

Gly Glu Arg Arg Arg Lys Lys Arg
1               5


<210>  4
<211>  7
<212>  PRT
<213>  Influenza virus

<400>  4

Arg Glu Arg Arg Arg Lys Arg
1               5


<210>  5
<211>  5
<212>  PRT
<213>  Influenza virus

<400>  5

Lys Arg Arg Arg Arg
1               5

<210>  6
<211>  6
<212>  PRT
<213>  Influenza virus

<400>  6

Lys Lys Arg Glu Lys Arg
1               5

<210>  7
<211>  6
<212>  PRT
<213>  Influenza virus

<400>  7

Lys Lys Arg Arg Lys Arg
1               5

<210>  8
<211>  8
<212>  PRT
<213>  Influenza virus

<400>  8

Lys Lys Lys Lys Lys Lys Lys Arg
1               5

<210>  9
<211>  12
<212>  PRT
<213>  Influenza virus

<400>  9

His Lys Gln Leu Thr His His Met Arg Lys Lys Arg
1               5                   10

<210>  10
<211>  32
<212>  DNA
<213>  Artificial Sequence

<220>
<223>  PCR primer

<400>  10
accattgggg aatgccccaa atatgtgaaa tc                                    32

<210>  11
<211>  40
<212>  DNA
<213>  Artificial Sequence

<220>
<223>  PCR primer

<400>  11
cgacaagctt gaattcttat ctcttttttc ttcttctctc                              40


<210>  12
<211>  39
<212>  DNA
<213>  Artificial Sequence

<220>
<223>  PCR primer

<400>  12
cgagggatcc gaattcagta aaggagaaga acttttcac                              39


<210>  13
<211>  39
<212>  DNA
<213>  Artificial Sequence

<220>
<223>  PCR primer

<400>  13
gcattcccca atggttttgt atagttcatc catgccatg                              39


<210>  14
<211>  18
<212>  PRT
<213>  Influenza virus

<400>  14

Cys Thr Gly Leu Arg Asn Ser Pro Gln Arg Glu Arg Arg Arg Arg Lys
1               5                   10                  15


Lys Arg